# EUROPEAN PATENT APPLICATION

(11) **EP 2 058 663 A1**
(43) Date of publication of application: **13.05.2009**
(21) Application number: 07021878.9
(22) Date of filing: 12.11.2007
(51) Int. Cl.: G01N 33/564

(54) **Method for the immobilization of an analyte on a solid support**

(71) Applicant: Euro-Diagnostica B.V., 6827 BN Arnhem (NL)
(72) Inventor: Van boekel, Martinus Adrianus Maria, 5388 CN Nistelrode (NL); Verheijen, Ronald, 6611 CE Overasselt (NL); Salden, Martinus Hubertus Leonardus, 6536 CL Nijmegen (NL)
(74) Representative: Habets, Winand

(57) **Abstract**

This invention is in the field of diagnostic assays, more in particular immunoassays wherein a first binding member is immobilized on a solid support in order to capture a second binding member. The invention relates to a method for the immobilization of an analyte on a solid support wherein the analyte is covalently attached to a biotin molecule to obtain a biotinylated analyte and wherein the biotinylated analyte is subsequently contacted with a high affinity binding member to form an analyte complex where after the analyte complex is contacted with the solid support.

## Description

### Field of the invention

This invention is in the field of diagnostic assays, more in particular immunoassays wherein a first binding member is immobilized on a solid support in order to capture a second binding member.

### Background art

One of the main goals of investigators in the fields of (clinical) diagnostics and contamination control programs consists of providing new analytical strategies to obtain accurate data as fast as possible. When a new screening test is required for commercial purposes, the method has to be sensitive, specific, fast, cheap and easy to perform. In general, immunological techniques (immunoassays) can fulfill these requirements to a great extent.

Over the past decade, single-use lateral flow immunoassays have been extremely successful both in the laboratory, outpatient clinic and primary care environments. In this type of assay all reaction components are impregnated or immobilised on a porous solid phase, usually a nitrocellulose membrane, and are brought into contact with the sample in sequence after addition of a diluent (Zuk RF, Ginsberg VK, Houts T et al. (1985) Clin Chem 31: 1144-1150, Bunce RA, Thorpe GH, Keen L (1991) Anal Chim Acta 249: 263-269, and May K (1994) In: D Wild (ed): The Immunoassay Handbook. Macmillan Press, London, 233-235).

This immunoassay format is also known as strip test, one step strip test, immunochromatographic test, rapid flow diagnostic, rapid immunoassay (test), lateral flow immunoassay (LFI), on site test (assay) or near patient test (NPT).

Immunoassays are analytical measurement systems that use antibodies as test reagents. The antibodies are usually attached to some kind of label and then used as reagents to detect the substance of interest. This is usually referred to as the conjugate. The label can be either an enzyme for colorimetric detection or a colored colloidal particle such as gold (Leuvering JHW, Thal PJHM, Van der Waart M, Schuurs AHWM (1980) J Immunoassay 1(1): 77-91), carbon, silica or latex for direct visualization of the immunoreaction. In practice, colloidal gold particles or gold nanoclusters having a diameter of 25-40 nm are probably the most commonly applied labels in lateral flow immunoassays (Verheijen, R., in: Analytical Biotechnology 2002, Birkhauser Verlag Ed: T. Schalkhammer p134-166).

A typical strip test device consists of a test strip that is mounted in a plastic cassette (housing). In a commercial setting, the test strip may be made up of a number of components, including a sample pad, a conjugate pad, an absorbent pad and a membrane that contains the capture reagents. The main purpose of the housing is to fixate the several components of the test strip and to keep them in close contact with each other. Moreover, the housing determines the dimensions of the sample well and contains the viewing window with readout indications.

When describing the principle of the lateral flow immunoassay, one has to distinguish between the direct assay that is usually used to detect high molecular mass components, usually proteins, and the indirect or competitive assay usually used to detect low molecular mass analytes such as drug residues, antibiotics, hormones, etc. In both types of tests, the user dispenses a liquid sample (buffer extract, milk, urine, serum, plasma, whole blood, etc) on to the sample pad. In a typical assay, the sample then flows through the sample pad into the conjugate pad, where it releases and mixes with the detector reagent. Other configurations are also known in the art, for instance where the sample is first contacted with the detection region and only subsequently the conjugate is then contacted with the detection region (Verheijen, R., in: Analytical Biotechnology 2002, Birkhauser Verlag Ed: T. Schalkhammer p134-166).

Lateral-flow assays may require a relatively large sample size in order to allow for the flow of the sample and label or conjugate particles that is characteristic of the lateral flow assay. More particularly, currently available lateral flow assays employ conjugates that are located downstream from a sample deposition point and upstream from a detection zone. The sample itself is relied upon to re-suspend the conjugates and carry them to the detection zone. Alternatively, additional diluent can be added with the sample to carry the sample to the conjugate pad, aid in re-suspending the conjugate particles, and then reach the detection zone. In either case, the sample addition is typically applied upstream from the conjugate particles to aid in the re-suspension of the particles. Note that "upstream" and "downstream" refer to the position of an item relative to the direction of flow of a sample on the assay device.

In the direct assay format for detecting high molecular mass analytes such as proteins, the test detector reagent may consist of gold nanoclusters coated with specific antibodies to the protein of interest. When that particular protein is present in the sample it will react with the test detector reagent. The formed protein-antibody-gold complexes are mobile and able to move freely from the reagent pad into the membrane with the flow of the fluid. At the test line, the complexes will be captured by immobilized anti-protein antibodies. Thus, the presence of the protein of interest in the sample will result in a colored test line. The color intensity of the test line may be proportional to the concentration of the analyte in the sample. When the concentration of the protein of interest is lower than the lowest detection concentration or when the protein of interest is completely absence, no test line will be visible. Excess sample that flows beyond the test and control lines is taken up in the absorbent pad (Verheijen, R., in: Analytical Biotechnology 2002, Birkhauser Verlag Ed: T. Schalkhammer, p134-166).

In the competitive assay format for detecting low molecular mass analytes, the test detector reagent consists of gold nanoclusters coated with antibodies to the analyte of interest. The test line here consists of a protein-analyte conjugate. The more analyte present in the sample, the more effectively it will compete with the immobilised analyte on the membrane for binding to the limited amount of antibodies of the detector reagent. Thus, the absence of the analyte in the sample will result in a colored test line, whereas an increase in the amount of analyte will result in a decrease of signal in the readout zone. At a certain concentration of analyte in the sample, the test line will be no longer visible. The lower detection limit is defined as the amount of analyte in the sample that just causes total invisiblity of the test capture line.

Strip test devices are commercially available for an increasing number of antigens (high- and low-molecular mass). The first major target analyte for this test format was human chorionic gonadotropin (HCG) for the detection of pregnancy. At present, a great variety of test strip assays are available (Anonymous, Syllabus of a two-day seminar on Solid Phase Membrane-Based Immunoassays, Paris, September 25-26th, 1997, Millipore Corporation, Bedford, MA, USA, Anonymous, Syllabus of The Latex Course, London, October 1-3th, 1997, Organised by Bangs Laboratories, Inc., Fishers, IN, USA, Price CP, Thorpe GHG, Hall J, Bunce RA (1997) In: CP Price, DJ Newman (eds): Principles and Practices of Immunoassays (2nd edition). MacMillan Reference Ltd, London, 579-603, Hobbs FDR, Delaney BC, Fitzmaurice DA et al (1997). Health Technology Assessment 1(5)). Examples of commercially available lateral flow immunoassays are e.g. immunoassays for the detection of hormones (pregnancy, fertility, ovulation, menopause, sexual disorder, thyroid functions), tumour markers (prostate, colorectal, etc.). viruses (HIV, Hepatitis B and C), bacteria (Streptococcus A and B, Chlamydia trachomatis, Treponema pallidum, Heliobacter pylori, etc.), IgE (allergy) and troponin T in cardiac monitoring. All these analytes are measured on the basis of their presence or absence. An extensive review of near patient testing in primary care has been published by Hobbs et al., vide supra.

Although strip tests appear simple, complex interactions among their various components lead to a number of challenges in both the development and manufacturing environments. These challenges are even greater for quantitative tests where the color intensity of the test line must be repeatable between production lots. From a developmental perspective, creating a successful test system means optimizing the interactions between its raw materials, component design, and manufacuring techniques.

A typical dilemma common to every lateral flow immunoassay is that it is desired that the mobile phase can move through the membrane with as less as possible hindrance from the membrane whereas the immobile phase should be irremovably attached to the membrane. The pore size of the membrane determines the flow rates of the mobile phase. For fast assays, a fast flowing membrane is preferred and such membranes are characterized by relatively large pore sizes. It is a problem for such membranes to retain analytes, in particular small peptides in the immobile phase.

Nitrocellulose is probably the most commonly used polymer for strip test membranes. The pore size of such membranes varies between 5 to 20 µm, which is large compared to a pore size of 0.2 to 1.2 µm of a nitrocellulose membrane used for protein blotting. A large pore size implies a small membrane surface area and consequently a low protein binding capacity, i.e. 20-30 µg IgG/cm² instead of the 110 µg IgG/cm² for a blotting membrane with a pore size of 0.45 µm.

Many lateral flow assays have been described in the art and methods for the immobilization of first binding members, most notably antigens or antibodies, are known in the art. In general, methods for immobilizing small peptides employ the principle of conjugating the small peptide to a larger molecule such as for instance a larger peptide, protein or sugar. It has also been described to use fusion proteins, commonly produced in recombinant organisms.

A method well-known in the art is coupling of an analyte such as a small peptide to larger proteins such as bovine serum albumine (BSA) or ovalbumin (Verheijen, R., in: Analytical Biotechnology 2002, Sirkhauser Verlag Ed: T. Schalkhammer, p134-166). Other known methods are based on the capturing of an analyte to a solid support wherein the high affinity binding of biotin and streptavidin is employed. In those prior art methods, avidin or streptavidin is first immobilized on a solid support and used to capture a biotinylated analyte such as a peptide.

One common method for biochemical analyte capture involves coupling analytes to the vitamin biotin in a process termed biotinylation. Biotin binds strongly to the proteins avidin and streptavidin, and biotionylated molecules are easily captured by a surface having immobilized avidin or streptavidin molecules. The biotin-avidin and biotinstreptavidin complexes have extremely large association constants (Kₐ =10¹⁵ M⁻¹ for avidin and10¹³ M⁻¹ for streptavidin), energetically equivalent to covalent bonds, and are stable over a wide range of temperature and pH. For these reasons, biotin is commonly employed for analyte capture.

Biotinylation is typically accomplished using a chemically active form of biotin to label exposed lysine residues on target proteins. Lysine is one of the most frequently occurring amino acids, and chemical biotinylation can, therefore, be used to biotinylate and capture essentially all proteins.

Biotin/streptavidin interactions have been utilized in other ways in immunoassay procedures for some time. For example, in U.S. 5,126,241, streptavidin adsorbed to solid support is used to bind biotinylated antigen in a procedure which involves incubation to form a complex in which the analyte to be determined competes with label and solid support for access to an antibody capable of binding all three. U.S. 4,496,654 describes an assay for human chorionic gonadotropin conducted by binding biotinylated antibody to an avidin-coupled paper disk, reacting the antibody on a disk with a solution suspected of containing hCG, and then determining the amount of hCG on the disk using standard determination techniques.This assay results in a sandwich of hCG formed from anti- hCG bound to solid support through biotin/avidin linkage and labeled anti-hCG. This assay does not involve a lateral flow of sample.

U.S. 5,001,049 describes a method for determining antibodies against human HIV which involves incubating streptavidin-derivatized solid support with a biotinylated peptide reactive with anti-HIV, and then detecting any bound antibody with labeled antibody receptor. Again, lateral flow does not take place in these assays. RE 34,132, which is a reissue of Patent Number 4,945,042, describes a direct assay for an antibody wherein the antibody to be determined serves as a link between a labeled epitope and an epitope bound to substrate through a streptavidin/biotin link. Again, speed of reaction is not critical, since a lateral flow format is not required.

It is the subject of this application to provide at least an alternative immobilization technology, preferably an immobilization technology with advantages over the prior art. Such advantages may be found in the specificity, sensitivity, costs or ease of manufacturing of the immunoassays and or in advantages in costs or ease of handling of the eventual diagnostic assay itself.

### Summary of the invention

Surprisingly it was found that an improved specificity and or sensitivity could be achieved when an analyte such as an antigen or antibody was immobilized on a membrane while conjugated to biotin and a high affinity binding member such as avidin, streptavidin, neutravidin or streptavidin-hydrazide before immobilizing the thus obtained complex on the solid support.

The invention therefore relates to a method for the immobilization of an analyte on a solid support wherein the analyte is covalently attached to a biotin molecule to obtain a biotinylated analyte and wherein the biotinylated analyte is subsequently contacted with a high affinity binding member to form an analyte complex where after the analyte complex is contacted with the solid support.

### Detailed description of the invention

It was found that a method wherein an analyte was labeled with biotin and then conjugated to a high affinity binding member such as streptavidin, avidin, neutravidin or streptavidin-hydrazide before immobilizing the thus obtained complex to a solid support, yielded particular advantages over the known immobilization techniques.

The invention therefore relates to a method for the immobilization of an analyte on a solid support wherein the analyte is covalently attached to a biotin molecule to obtain a biotinylated analyte and wherein the biotinylated analyte is first contacted with a high affinity binding member to form an analyte complex whereafter the analyte complex is contacted with the solide support.

A high affinity binding member in this context is to be interpreted as a molecule capable of binding to biotin with an affinity constant comparable to a covalent bond. The skilled person will recognize this to be in the order of Kₐ =10¹⁵ M⁻¹ to 10¹¹ M⁻¹, typically 10¹³ M⁻¹ for streptavidin.

The high affinity binding member may advantageously be selected from the group consisting of avidin, streptavidin, neutravidin or streptavidin-hydrazide

It is to be understood that this method differs from the prior art methods described above in that the high affinity binding member is not coupled to the solid support when mixed with the biotinylated analyte; only after the formation of the bond between biotin and the high affinity binding member, the thus obtained complex is immobilized on the solid support. For the avoidance of doubt, the contacting of the biotinylated analyte with a high affinity binding member to form an analyte complex is preferably performed in solution or in any other form wherein neither the biotinylated analyte nor the high affinity binding member is attached to a solid support.

The term analyte in this context is to be interpreted as a molecule capable of binding to another molecule, in particular an antigen or antibody. Antigens may consist of a wide range of molecules such as haptens, sugars, peptides, proteins oligosaccharides and many more known by the skilled person. Antibodies may be monoclonal or polyclonal antibodies. Antibodies may be derived from human clinical samples or bodily fluids such as for instance saliva, sweat, sputum, urine, blood, plasma, serum, vaginal fluid or cerebrospinal fluid. They may also be derived from experimental animals, such as mice rats, rabbits, lama's, camels or from recombinant sources such as bacteria, yeasts or human cells.

The method may advantageously be employed when the analyte consists of small molecules. In this context, the term small molecule is to be interpreted as meaning a molecule with a molecular mass below 100,000 (hundred thousand) Da, preferably below 50,000 Da, such as 40,000, 30,000, 20,000 or 10,000 Da. Particularly advantages are obtained when the small molecule is even smaller than 10,000 Da, such as for instance smaller than 8000, 6000, 4000, 2000 or 1000 Da. Examples of such small molecules may be antibody fragments, synthetic of natural peptides or haptens.

Alternatively, the term small molecules may also be functionally defined, for instance as a function of the pore size of the solid support such as a membrane. It is known in the art that the adhesion or binding of molecules to solid supports depends on the pore size of the solid support. If the pore size is bigger, binding of an analyte such as a peptide to the solid support will be poorer than the binding to a solid support with smaller pores. Thus, a small molecule may also be defined as a molecule that will not or not detectably stick to a given membrane with a particular pore size.

Particularly advantageous results were obtained when the small molecules were peptides. In this study, peptides were used in the range of 5 to 100 amino acids, such as for instance 10, 12, 14, 16, 19, 20, 30, 50 or 80 amino acids. Such peptides are know for their poor binding to solid supports, in particular porous membranes, more in particular porous membranes with large pore sizes. When such peptides were coupled to biotin and used in the method according to the invention, improved sensitivity and specificity of the resulting assay was observed. Moreover, the method of manufacturing was more easy and less labor intensive as compared with the prior art methods. In addition, the resulting lines of positive samples at the detection region was more sharp and clear when compared to prior art methods.

The term solid support in this respect is to be interpreted as any support or surface suitable for performing an immunoassay. In particular the method employs a solid support that is porous to allow a capillary flow through the material. Examples of such porous solid supports are nitrocellulose membranes.

Methods for covalently attaching a biotin molecule to any small molecule are known in the art, and kits for biotinylation are commercially available, for instance from Roche Diagnostics, Indianapolis, Cat No. 11008960 001. The skilled person will know how to handle and bind with a high affinity binding member such as avidin, streptavidin, neutravidin or streptavidin-hydrazide to the biotinylated analyte.

It was tried to bind synthetic peptides directly to nitrocellulose membranes Millipore HF075, HF090, HF120, HF135, HF180 or HF240. For that purpose, the following synthetic 12-mer peptides as described in WO 03/050542 were spotted on the membranes and dried.

| | |
|---|---|
| 0002-27 | H Q K R G Cit G W S R A A |
| 0002-29 | H Q R R V Cit G W S R A A |
| 0002-31 | H Q R R T Cit G G S R A A |
| 0002-32 | H Q R K W Cit G A S R A A |
| 0002-36 | H Q F R F Cit G Cit S R A A |
| 0002-37 | H Q K W R Cit G R S Cit A A |
| 0002-63 | H Q F R F Cit G W S R A A |
| 0107-32 | K P Y T V Cit K F M R R P |
| 0107-35 | A R F Q M Cit H Cit R L I R |
| 0107-45 | Y S F V W Cit S H A R P R |
| 0113-30 | A R F Q M R H Cit R L I R |
| 0218-36 | R N L R L Cit R E R N H A |

Herein "Cit" depicts citrulline and the other amino acids are shown in the one-letter code for amino acids. In addition, the cyclic variants of these peptides (example 1) were also tested in the same setting.

These linear and cyclic peptides are known for their excellent reactivity with antibodies in the sera from patients suffering from Rheumatoid Arthritis. The dried membranes were tested for reactivity with 20 of such sera ranging from low to very high titers of antibody and it appeared that none of the peptides were retained on the membranes, which was evident by the fact that none of the lines where the peptides were applied gave a significant reaction with the antibodies.

The same peptides were then biotinylated (example 2) and tested for reactivity in the same manner as described above. Again, no reaction was observed, which was attributed to the fact that the biotinylated peptides were again too small to be retained by the membranes. In the examples section the results are shown obtained with the reference panel of high, medium and low plasma samples only.

In order to provide for a comparative example, a prior art method was used to immobilize the peptides more effectively. Therefore, a conjugate was prepared between the peptides and bovine serum albumin (BSA) and that conjugate was spotted onto nitrocellulose. Some false positive results were obtained as well as false negatives. In total this was rated as an average result (Table 1).

Next, the biotinylated peptides were reacted with anti-biotin polyclonal antibodies (Jacksons laboratories, product 200-002-096 IgG fraction) and then spotted onto the membranes. Although this somewhat improved the sensitivity of the assay, the same moderate specificity was observed as with the prior art method employing a peptide-BSA conjugate (Tables 2 and 3).

When the biotinylated peptides were mixed with a high affinity binding member such as avidin, streptavidin, neutravidin or streptavidin-hydrazide before immobilizing the analyte complex on the membranes, the results greatly improved. Less false positives were observed in comparison to any of the above methods and good specificity was obtained. In particular streptavidin worked very well, this resulted in an assay with 100% specificity and 100% sensitivity (Tables 2 and 3).

In the examples, several peptides were tested for their reactivity with antibodies from patient with Rheumatoid Arthritis (RA). It was shown that in all cases an improved reactivity was observed in comparison to prior art methods. Improved reactivity in this sense is to be interpreted as an improved sensitivity or specificity of the assay or both. The invention therefore also relates to a method as described above wherein the analyte is an antigen reactive with antibodies obtained from patients suffering from Rheumatoid Arthritis.

Improved reactivity with RA antibodies was observed when a cyclic citrullinated peptide was used as the analyte (Table 3).

The method according to the invention may be employed with a wide range of solid supports. Advantageous results were obtained when the solid support was selected from the group consisting of fast flowing membranes such as Millipore HF075, HF090, HF120, HF135, HF180 or HF240 or membranes with comparable flow properties.

The invention also relates to a solid support with an analyte complex immobilized thereon, obtainable by the process according to the invention. Such a solid support may be used for instance in a lateral flow assay for the detection of antibodies or antigens, such as detection of antibodies specific for Rheumatoid Arthritis. In such a lateral flow assay a cyclic ritrullinated peptide is advantageously immobilized on the solid support.

In the examples it is described how several analyte complexes were contacted with the solid support. This was done with the help of a machine which is not to be interpreted as that this is mandatory. A skilled person will be aware of other options for depositing an analyte complex onto a solid support, including but not limited to dipping spraying, blotting, spotting and many others.

### Examples

### Example 1: Peptides

The following linear peptides were obtained commercially from Polypeptide Laboratories, 365 Maple Avenue, Torrance CA 90503.

| | |
|---|---|
| 2-27 | H Q K R G Cit G W S R A A |
| 2-29 | H Q R R V Cit G W S R A A |
| 2-31 | H Q R R T Cit G G S R A A |
| 2-32 | H Q R K W Cit G A S R A A |
| 2-36 | H Q F R F Cit G Cit S R A A |
| 2-37 | H Q K W R Cit G R S Cit A A |
| 2-63 | H Q F R F Cit G W S R A A |
| 107-32 | K P Y T V Cit K F M R R P |
| 107-35 | A R F Q M Cit H Cit R L I R |
| 107-45 | Y S F V W Cit S H A R P R |
| 113-30 | A R F Q M R H Cit R L I R |
| 218-36 | R N L R L Cit R E R N H A |

Cyclic variants of these peptides were also obtained commercially from Polypeptide Laboratories, 365 Maple Avenue, Torrance CA 90503. These peptides were cyclisized through their cysteine residues and had the following primary sequence:

| | |
|---|---|
| Cyclic 2-27 | G S Q H C H Q K R G Cit G W S R A A C G-NH2 |
| Cyclic 2-29 | G S Q H C H Q R R V Cit G W S R A A C G-NH2 |
| Cyclic 2-31 | G S Q H C H Q R R T Cit G G S R A A C G-NH2 |
| Cyclic 2-32 | G S Q H C H Q R K W Cit G A S R A A C G-NH2 |
| Cyclic 2-36 | G S Q H C H Q F R F Cit G Cit S R A A C G-NH2 |
| Cyclic 2-37 | G S Q H C H Q K W R Cit G R S Cit A A C G-NH2 |
| Cyclic 2-63 | G S Q H C H Q F R F Cit G W S R A A C G-NH2 |
| Cyclic 107-32 | G S Q H C K P Y T V Cit K F M R R P C G-NH2 |
| Cyclic 107-35 | G S Q H CAR F Q M Cit H Cit R L I R C G-NH2 |
| Cyclic 107-45 | G S Q H C Y S F V W Cit S H A R P R C G-NH2 |
| Cyclic 113-30 | G S Q H CAR F Q M R H Cit R L I R C G-NH2 |
| Cyclic 218-36 | G S Q H C R N L R L Cit R E R N H A C G-NH2 |

The C-terminus of the peptides was amidated.

### Prior art example: Coupling of peptides to BSA

BSA-peptide complexes were prepared by cross-linking BSA to the linear and cyclic peptides as shown in example 1. For that purpose, the peptides were synthesized wherein a cysteine residue was added to the N-terminal side of the peptides shown in example 1. Cross-linking of BSA was achieved using the cross-linker sulfo-SMCC obtained from Pierce, Meridian Road Rockford IL, product nr 22322 according to the manufacturer's protocol. In essence: BSA was activated when dissolved at 1mg/ml in PBS and 20 ug crosslinker was added per mg BSA. The mixture was incubated at room temperature for 1 hour and the reaction was blocked with 1mM Tris-WCI, pH 7.4 final concentration. Free cross-linker was removed with a PD1O column. Peptides were added at 1 mg per mg of activated BSA and incubated for 2 hours at room temperature. Free peptides were removed by dialysis against PBS pH 7.4.

Polyacrylamide gel analysis revealed a molecular weight of 100kDa for the BSA-peptide complex, meaning that on average about 13 peptide molecules were attached to one BSA molecule.

The following peptides were obtained.

| | |
|---|---|
| BSA 2-27 | BSA C H Q K R G Cit G W S R A A |
| BSA2-29 | BSA C H Q R R V Cit G W S R A A |
| BSA2-31 | BSA C H Q R R T Cit G G S R A A |
| BSA2-32 | BSA C H Q R K W Cit G A S R A A |
| BSA 2-36 | BSA C H Q F R F Cit G Cit S R A A |
| BSA 2-37 | BSA C H Q K W R Cit G R S Cit A A |
| BSA2-63 | BSA C H Q F R F Cit G W S R A A |
| BSA 107-32 | BSA C K P Y T V Cit K F M R R P |
| BSA 107-35 | BSA C A R F Q M Cit H CR R L I R |
| BSA 107-45 | BSA C Y S F V W Cit S H A R P R |
| BSA 113-30 | BSA C A R F Q M R H Cit R L I R |
| BSA218-36 | BSA C R N L R L Cit R E R N H A |

### Example 2: Biotinylation of analytes

The set of linear and cyclic peptides shown in Example 1 were also obtained Polypeptide Laboratories, 365 Maple Avenue, Torrance CA 90503 with an N-terminal biotin residue, These peptides had the following primary structure:

| | |
|---|---|
| Biotin 2-27 | Biotin H Q K R G Cit G W S R A A |
| Biotin 2-29 | Biotin H Q R R V Cit G W S R A A |
| Biotin 2-31 | Biotin H Q R R T Cit G G S R A A |
| Biotin 2-32 | Biotin H Q R K W Cit G A S R A A |
| Biotin 2-36 | Biotin H Q F R F Cit G Cit S R A A |
| Biotin 2-37 | Biotin H Q K W R Cit G R S Cit A A |
| Biotin 2-63 | Biotin H Q F R F Cit G W S R A A |
| Biotin 107-32 | Biotin K P Y T V Cit K F M R R P |
| Biotin 107-35 | Biotin A R F Q M Cit H Cit R L I R |
| Biotin 107-45 | Biotin Y S F V W Cit S H A R P R |
| Biotin 113-30 | Biotin A R F Q M R H Cit R L I R |
| Biotin 218-36 | Biotin R N L R L Cit R E R N H A |

### Biotinylated and Cyclic:

| | |
|---|---|
| Biotin-Cyclic 2-27 | Biotin G S Q H C H Q K R G Cit G W S R A A C G-NH2 |
| Biotin-Cyclic 2-29 | Biotin G S Q H C H Q R R V Cit G W S R A A C G-NH2 |
| Biotin-Cyclic 2-31 | Biotin G S Q H C H Q R R T Cit G G S R A A C G-NH2 |
| Biotin-Cyclic 2-32 | Biotin G S Q H C H Q R K W Cit G A S R A A C G-NH2 |
| Biotin-Cyclic 2-36 | Biotin G S Q H C H Q F R F Cit G Cit S R A A C G-NH2 |
| Biotin-Cyclic 2-37 | Biotin G S Q H C H Q K W R Cit G R S Cit A A C G-NH2 |
| Biotin-Cyclic 2-63 | Biotin G S Q H C H Q F R F Cit G W S R A A C G-NH2 |
| Biotin-Cyclic 107-32 | Biotin G S Q H C K P Y T V Cit K F M R R P C G-NH2 |
| Biotin-Cyclic 107-35 | Biotin G S Q H C A R F Q M Cit H Cit R L I R C G-NH2 |
| Biotin-Cyclic 107-45 | Biotin G S Q H C Y S F V W Cit S H A R P R C G-NH2 |
| Biotin-Cyclic 113-30 | Biotin G S Q H C A R F Q M R H Cit R L I R C G-NH2 |
| Biotin-Cyclic 218-36 | Biotin G S Q H C R N L R L Cit R E R N H A C G-NH2 |

### Example 3: Preparation of analyte complexes using avidin, streptavidin, neutravidin or streptavidin-hydrazide.

Peptides as described above were dissolved in Phosphate Buffered Saline (PBS) at a concentration of 1 mg/ml. They were mixed with a 1 mg/ml solutions of either avidin, streptavidin, neutravidin or streptavidin-hydrazide in PBS in a molar ratio of peptide: avidin, streptavidin, neutravidin or streptavidin-hydrazide of 5 : 2. The mixture was incubated at room temperature for 10 minutes and used immediately. Avidin, streptavidin, neutravidin and streptavidin-hydrazide were obtained from Pierce.

In a comparative experiment wherein the peptides were mixed with avidin, streptavidin, neutravidin or streptavidin-hydrazide in molar ratios of 1:1, 2:1, 4:1 and 5:2 and tested with a limited set of RA antibodies. The 5:2 molar ratio appeared to be optimal. The other ratios still yielded acceptable results, it is therefore concluded that the ratio is not critical and can be optimized for each individual analyte complex. In general it will be advantageous to use a molar excess of peptide or protein.

### Example 4: Preparation of analyte complexes using monoclonal anti-biotin antibodies.

Biotinylated peptides as described above were dissolved in Phosphate Buffered Saline (PBS) at a concentration of 1 mg/ml. They were mixed with a 1.3 mg/ml solution of monoclonal anti-biotin antibody obtained from Jacksons laboratories, product 200-002-096 IgG fraction. The mixture was incubated at 37°C for 30 minutes and used immediately.

### Example 5: immobilization of analyte complexes on a solid support.

Analyte complexes were spotted in the form of a thin line on a solid support using a Kinematic 1600 machine. Solid supports used were Millipore HF075, HF090, HF120, HF135, HF180 and HF240 membranes. The stripe rate was 0.9 ul/cm at a speed of 10 cm/sec. Membranes were blocked using a PBS solution containing 1% Bovine Serum Albumin (BSA) and 1% Pluronic F68, a commercially available surfactant. For that purpose, the entire strip was sprayed using a Biodot XYZ 3000 machine equipped with an air jet dispensing head using the following settings: bed speed 5 cm/sec, dispenser 10 ul/sec, y axis 20 and micrometer 0,0. The membranes were dried in a dry room at room temperature (21 °C) for 24 hours and cut into strips of 5mm width. The length of the strips was about 30 mm and the analyte complexes were striped at about 7 mm from the top. The results obtained with the various membrane types were essentially the same. HF 180 membranes provided an optimal mix of flow properties and sensitivity/specificity. Those results are shown in tables 1 - 3, the results obtained with the other membranes were comparable if not identical.

### Example 6: Performing a lateral flow assay

An absorbent pad was attached to the top of the strip and the bottom of the strip was placed in an Eppendorf reaction vessel of 1.5 ml containing 75 ul of antibody solution. The antibody solution was allowed to fully migrate into the strip. The strip was then placed in another vessel containing 75 ul of a conjugate solution and a fluid stream was allowed to reach the absorbent pad. This was allowed to carry on for 20 minutes at room temperature.

Antibody solutions were prepared by mixing 15 ul human plasma with 60 ul PBS/ 1% BSA. Four different human plasma samples were used:
a) Normal Human plasma, non-reactive
b) Low titer Rheumatoid Arthritis plasma
c) Medium titer Rheumatoid Arthritis plasma
d) High titer Rheumatoid Arthritis plasma.

The plasma samples were derived from a master batch obtained from Trina International Nanikon in Switzerland. Cat. Nr DA 1708. High titer plasma was prepared by diluting the master batch plasma in normal human plasma until a titer of 1600 Units/ml was obtained when measured in the EuroDiagnostica Immunoscan RA anti CCP Test Kit; EuroDiagnostica BV Amhem, The Netherlands, Cat No RA-96RT. Medium titer plasma was obtained by diluting the High titer plasma until a titer of 400 Units/ml was obtained, the low titer plasma was obtained by further dilution in normal human plasma until a titer of 25 Units/ml was obtained.

Conjugate solution was prepared by mixing 30 ul of a gold conjugate obtained from British Biocell International (BBI); anti-human IgG gold conjugate Cat. No: BA.GAHL 40 with 70 ul running buffer. Running buffer used consisted of PBS with 1% BSA.

The color intensity of the line on each device was determined using a visual scale ("Rann scale") ranging from 0-11, wherein 0 represents no color and 11 represents the most intense color. The RANN scale is used as a comparator, where the test signal intensity is compared to the equivalent RANN scales intensity and assigned a value for result recording purposes. The RANN score card consists of 5 lines with varying intensity ranging from very faint to very intense. The extremes on the card correspond to line intensities of 1 and 10. If the intensity of a test line is below the least intensive line on the score card the intensity of the test line is scored as 0. If the intensity of a test line equals the least intensive line on the score card, the intensity of the test line is scored as 1. If the intensity of a test line is in between the least intensive line and the next intensive line on the score card, the intensity of the test line is scored as 2 and so on. Maximum score obtainable was 11 meaning more intense than the most intense line on the RANN score card.

The intensity the stripes in each test zone that was obtained with the materials as described above is summarized in Tables 1 - 3.

**Table 1: RANN Score of test lines obtained with the various analytes and analyte complexes according to the prior art.**

| **Peptide** | **linear** | **cyclic** | **Biotin Linear** | **Biotin Cyclic** | **BSA Linear** | **BSA Cyclic** |
|---|---|---|---|---|---|---|
| 2-27 | 0/0/0/0 | 0/0/0/0 | 0/0/0/0 | 0/0/0/1 | 0/0/1/4 | 1/0/0/4 |
| 2-29 | 0/0/0/0 | 0/0/0/0 | 0/0/0/0 | 0/0/0/0 | 0/1/2/4 | 2/4/4/4 |
| 2-31 | 0/0/0/0 | 0/0/0/0 | 0/0/0/0 | 0/0/0/0 | 3/3/3/4 | 2/2/5/5 |
| 2-32 | 0/0/0/1 | 0/0/0/0 | 0/0/0/0 | 0/0/0/0 | 0/0/0/0 | 2/2/2/4 |
| 2-36 | 0/0/0/0 | 0/0/0/0 | 0/0/0/0 | 0/0/0/0 | 0/0/2/3 | 0/0/3/3 |
| 2-37 | 0/0/0/0 | 0/0/0/1 | 0/0/0/0 | 0/0/0/0 | 2/2/3/3 | 1/0/0/0 |
| 2-63 | 0/0/0/0 | 0/0/0/0 | 0/0/0/0 | 0/0/0/0 | 0/0/0/2 | 0/0/2/4 |
| 107-32 | 0/0/0/0 | 0/0/0/0 | 0/0/0/1 | 1/0/0/0 | 0/0/0/0 | 0/0/0/3 |
| 107-35 | 0/0/0/0 | 0/0/0/0 | 0/0/0/0 | 0/0/0/0 | 0/0/0/4 | 0/0/0/0 |
| 107-45 | 0/0/0/0 | 2/0/0/0 | 0/0/0/0 | 0/0/0/0 | 2/0/0/3 | 1/0/0/0 |
| 113-30 | 0/0/0/0 | 0/0/0/0 | 0/0/0/1 | 0/0/0/0 | 0/0/0/3 | 0/0/2/4 |
| 218-36 | 0/0/0/0 | 0/0/0/0 | 1/0/0/0 | 0/0/0/0 | 1/0/0/0 | 0/0/0/0 |

| | | | | | | |
|---|---|---|---|---|---|---|
| Table 1: RANN score of normal human serum/low titer/medium titer/high titer plasma samples obtained with test strips carrying the analytes and analyte complexes as indicated. | | | | | | |

**Table 2: RANN Score of test lines obtained with the various analytes and analyte complexes according to the invention in comparison to the prior art linear BSA conjugates. Results with Linear Peptides are shown**

| **Peptide** | **BSA Linear** | **Avidin-biotin-peptide** | **Streptavidin-biotin-peptide** | **Neutravidin-biotin-peptide** | **Streptavidin-hydrazide-biotin-peptide** | **Antibody-biotin-peptide** |
|---|---|---|---|---|---|---|
| 2-27 | 0/0/1/4 | 0/2/4/4 | 0/2/4/6 | 0/2/4/6 | 0/2/4/4 | 1/0/1/4 |
| 2-29 | 0/1/2/4 | 0/2/4/4 | 0/1/4/6 | 0/2/4/4 | 0/2/4/4 | 2/4/4/4 |
| 2-31 | 3/3/3/4 | 0/2/3/3 | 0/2/4/6 | 0/2/3/5 | 2/2/2/3 | 2/2/5/5 |
| 2-32 | 0/0/0/0 | 0/2/3/3 | 0/2/3/6 | 0/2/3/4 | 0/2/2/3 | 2/2/2/4 |
| 2-36 | 0/0/2/3 | 0/2/3/3 | 0/2/4/6 | 0/2/3/6 | 0/1/3/3 | 0/0/3/3 |
| 2-37 | 2/2/3/3 | 1/2/4/6 | 0/2/3/6 | 0/2/4/6 | 1/2/5/6 | 1/0/1/0 |
| 2-63 | 0/0/0/2 | 0/2/4/6 | 0/3/4/6 | 0/2/4/6 | 0/2/2/6 | 4/0/2/4 |
| 107-32 | 0/0/0/0 | 0/2/2/4 | 0/1/2/7 | 0/2/2/2 | 0/2/2/2 | 1/0/0/3 |
| 107-35 | 0/0/0/4 | 1/2/2/3 | 0/1/2/4 | 0/2/2/3 | 2/2/2/3 | 0/0/0/0 |
| 107-45 | 2/0/0/3 | 0/2/3/6 | 0/1/2/4 | 0/2/3/6 | 0/2/3/6 | 1/0/0/0 |
| 113-30 | 0/0/0/3 | 1/2/4/4 | 0/1/1/4 | 1/2/4/4 | 1/2/4/4 | 4/0/2/4 |
| 218-36 | 1/0/0/0 | 1/2/4/6 | 0/1/2/2 | 1/2/4/6 | 1/2/4/6 | 0/0/0/0 |

| | | | | | | |
|---|---|---|---|---|---|---|
| Table 2: RANN score of normal human serum/low titer/medium titer/high titer plasma samples obtained with test strips carrying the analytes and analyte complexes according to the invention as indicated. | | | | | | |

**Table 3: RANN Score of test lines obtained with the various analytes and analyte complexes according to the invention in comparison to the BSA conjugates. Results with Cyclic Peptides are shown.**

| **Peptide** | **BSA Cyclic** | **Avidin-biotin-peptide** | **Streptavidin-biotin-peptide** | **Neutravidin-biotin-peptide** | **Streptavidin-hydrazide-biotin-peptide** | **Antibody-biotin-peptide** |
|---|---|---|---|---|---|---|
| 2-27 | 1/0/0/4 | 0/2/4/7 | 0/2/6/11 | 0/2/4/8 | 1/2/6/8 | 1/0/0/8 |
| 2-29 | 2/4/4/4 | 0/2/4/7 | 0/1/6/10 | 0/2/4/8 | 0/2/6/6 | 3/4/4/6 |
| 2-31 | 2/2/5/5 | 0/0/5/6 | 0/2/6/9 | 0/2/3/7 | 0/2/6/8 | 2/2/5/8 |
| 2-32 | 2/2/2/4 | 0/2/5/6 | 0/2/6/9 | 0/2/3/6 | 1/215/7 | 3/2/2/4 |
| 2-36 | 0/0/3/3 | 0/2/5/8 | 0/2/5/9 | 1/2/3/7 | 0/1/7/7 | 3/0/3/3 |
| 2-37 | 1/0/0/0 | 0/2/5/9 | 0/2/5/9 | 0/2/4/8 | 1/2/5/8 | 1/0/8/8 |
| 2-63 | 0/0/2/4 | 0/2/5/9 | 0/2/7/11 | 0/2/4/9 | 0/2/2/8 | 4/0/8/6 |
| 107-32 | 0/0/0/3 | 0/2/2/6 | 0/1/4/9 | 0/2/2/9 | 0/2/8/8 | 1/2/6/6 |
| 107-35 | 0/0/0/0 | 1/2/2/7 | 0/1/4/9 | 0/2/2/9 | 1/2/3/5 | 0/2/2/7 |
| 107-45 | 1/0/0/0 | 0/0/3/7 | 0/1/2/8 | 0/2/3/9 | 0/2/3/8 | 1/3/4/6 |
| 113-30 | 0/0/2/4 | 0/2/418 | 0/1/3/8 | 0/2/4/6 | 1/2/4/8 | 4/0/2/4 |
| 218-36 | 0/0/0/0 | 1/2/4/7 | 0/1/4/6 | 0/2/4/6 | 1/2/4/8 | 0/2/2/6 |

| | | | | | | |
|---|---|---|---|---|---|---|
| Table 3: RANN score of normal human serum/low tiler/medium titer/high titer plasma samples obtained with test strips carrying the analytes and analyte complexes according to the invention as indicated. | | | | | | |

## Claims

1. Method for the immobilization of an analyte on a solid support wherein the analyte is covalently attached to a biotin molecule to obtain a biotinylated analyte and wherein the biotinylated analyte is first contacted with a high affinity binding member to form an analyte complex where after the analyte complex is contacted with the solid support

2. Method according to claim 1 wherein the high affinity binding member is a molecule selected from the group consisting of avidin, streptavidin, neutravidin and streptavidin-hydrazide

3. Method according to claims 1 or 2 wherein the analyte is a small molecule.

4. Method according to claims 1 -3 wherein the analyte is a peptide.

5. Method according to claim 1 -4 wherein the analyte is an antigen reactive with antibodies obtained from patients suffering from Rheumatoid Arthritis.

6. Method according to claim 5 wherein the analyte is a cyclic citrullinated peptide.

7. Method according to claims 1 to 6 wherein the solid support is a nitrocellulose membrane.

8. Method according to claim 7 wherein the solid support is a fast flowing membrane.

9. Method according to claim 8 wherein the membrane is selected from the group consisting of Millipore HF075, HF090, HF120, HF135, HF180 or HF240 or membranes with comparable or equivalent flow properties.

10. A solid support with an analyte complex immobilized thereon, obtainable by the method according to claims 1 to 9.

11. Lateral flow assay for the detection of antibodies or antigens comprising a solid support according to claim 10

12. Lateral flow assay according to claim 11 for the detection of antibodies specific for Rheumatoid Arthritis.

13. Lateral flow assay according to claim 12 wherein a cyclic citrullinated peptide is immobilized on the solid support.
